# EUROPEAN PATENT SPECIFICATION

(11) **EP 3 243 451 B1**
(45) Date of publication and mention of the grant of the patent: **05.08.2020**
(21) Application number: 16169300.7
(22) Date of filing: 12.05.2016
(51) Int. Cl.: A61B 17/12

(54) **DEVICE FOR ADJUSTABLY RESTRICTING INTRAVASCULAR FLOW**
VORRICHTUNG ZUR EINSTELLBAREN EINSCHRÄNKUNG VON INTRAVASKULÄREM STROM
DISPOSITIF DE RESTRICTION RÉGLABLE D'ÉCOULEMENT INTRAVASCULAIRE

(43) Date of publication of application: 15.11.2017
(73) Proprietor: IntelliStent AG, 6052 Hergiswil (CH)
(72) Inventor: Dovgaliuk, Arkadii, 04111 Kyiv (UA); Yemets, Illya, 04073 Kyiv (UA); Vogt, Paul Robert, 8835 Feusisberg (CH); Levis, Pierre, 8126 Zumikon (CH); Zaza, Beradze, 01015 Kyiv (UA)
(74) Representative: Schneider Feldmann AG Patent- und Markenanwälte

(56) References cited:
- EP-A1- 2 918 250
- US-A1- 2005 107 867
- US-A1- 2010 106 240

## Description

### FIELD OF THE INVENTION

The invention relates to a device for controlling the flow of a fluid like blood through a vessel, in particular for intravascular reduction of the vessel lumen. The device can be used for the treatment of medical conditions that require the reduction of blood flow and blood pressure distal to the site of device application.

### BACKGROUND OF THE INVENTION

Congenital heart disease with increased pulmonary blood flow requires stage-by-stage treatment when the risk of performing total surgical correction is high or impossible. The first stage in reducing pulmonary blood flow is surgical banding of the pulmonary artery. In case of dilated cardiomyopathy in children and adults this kind of operation will improve the functional status of the patient and significantly delay the necessity for heart transplantation. There is great need for this kind of operation because the number of patients is increasing every year. Nowadays, endovascular interventions can often substitute surgical treatment and are less traumatic. Nevertheless, pulmonary artery banding is still performed surgically. This is mainly due to the fact that the devices that were developed for endovascular pulmonary artery banding are bulky and need a large diameter of the delivery system, and thus, cannot be safely used in children.

Document US2010/0106240 discloses a medical device for controlling the flow of a fluid through a vessel comprising a stent part adapted to contact an inner surface of the vessel and a membrane part attached to the stent part, wherein the membrane part forms a flow opening the size of which is adjustable by means of a thread which extends along the flow opening wherein the membrane part is in the form of a tube with two openings at two opposing ends, wherein one of the two openings is the flow opening, wherein the two opposing ends comprise a first free end and a second end, wherein the flow opening is located at the first end and the second end is attached to the stent part, wherein the stent part is preferably tube-shaped and the membrane part is arranged within the stent part, and the size of the flow opening is adjustable by adjusting the size of the loop and wherein the size of the loop is adjustable by pulling on the thread.

### OBJECT OF THE INVENTION

It is an object of the present invention to provide a device for controlling the flow of a fluid through a vessel, in particular for adjustable intravascular flow restriction. Further objects and advantages of the invention are described hereinafter.

### DESCRIPTION OF THE INVENTION

The above mentioned object can be achieved by a device according to claim 1.

Disclosed is inter alia a medical device for controlling the flow of blood through a vessel comprising a stent, a membrane, a rigid member and a thread. The membrane is used to reduce the vessel lumen while the stent is preferably only used to fix the device in the vessel. Disclosed is also a method for controlling the flow of blood through a vessel using such a device wherein the membrane forms an opening and the thread forms a loop around said opening and by pulling at one end of the thread the loop is tightened and the size of the opening is reduced.

In the following, preferred embodiments of the invention are described. The features mentioned in respect of said embodiments are to be individually considered preferred features and they may be implemented individually or in any combination provided such features do not exclude each other.

Advantageously, by pulling on the thread the length of the free membrane edge and/or the diameter of the opening defined by said edge is reduced.

The present rigid member can have the function of centering the membrane (and/or the opening) in relation to the stent axis. The rigid member can also have the function of fixing and/or holding the thread. The member can for example be a tube.

For introducing the device into a vessel a stabilizing catheter can be used. Optionally, the thread can be pulled outwards through the stabilizing catheter.

It is preferred that after pulling on the thread the exposed part of the thread is cut off, preferably adjacent to the base of the stent. For cutting the thread a cutting catheter can for example be used. Optionally, the thread can be put between the turns of a spiral of such a catheter for the fixation of the thread during the cut off.

Of particular interest is a medical device for controlling the flow of a liquid through a vessel wherein the device comprises a stent part, a membrane part, a thread, and a rigid member. The membrane part is attached to the stent part and forms a flow opening (i.e. an opening for the fluid to pass through the device), wherein the size of the flow opening is adjustable by means of a thread which extends along and/or around and/or adjacent to the flow opening. Even though other solutions are possible it seems most useful if the thread forms a loop around the flow opening.

By pulling on the thread the length of the part of the thread that is arranged along and/or around and/or adjacent to the flow opening can preferably be reduced. Alternatively or in addition the length of the edge of the flow opening can preferably be reduced by pulling on the thread.

The device can be used as an alternative way to conventional surgical pulmonary artery banding. One of the advantages is that there is no necessity for general anesthesia and artificial lung ventilation. This can significantly reduce the time in the operating room, in the intensive care unit and in the hospital. Therefore, the costs will be significantly reduced.

Disclosed is also a method for the treatment of medical conditions that require the reduction of blood flow and/or the reduction of blood pressure, wherein the device disclosed in this document is used for this purpose. The device can be applied within a vessel to reduce the lumen of the vessel and thereby reduce blood flow past the device and/or pressure distal to the location of the device.

The membrane part is in the form of a tube with two openings at the two opposing ends. The two opposing ends comprise a first free and/or unattached end and a second end, wherein the second end is attached to the stent part.

Preferably, the first end of the membrane part is located downstream of the second end of the membrane part when the device is located inside the vessel.

When the flow opening is completely open the membrane part can be essentially cylindrical. When the size of the flow opening is reduced the membrane part can be funnel-shaped.

Preferably, the membrane part along its second end and/or along its outer circumference is sealably connected to the stent part to avoid fluid to pass between the membrane part and the stent part.

The membrane part further comprises a guide for the thread. The guide extends along the first end (preferably along and/or adjacent to the flow opening at the first end of the membrane part). From there the guide can preferably extend outwardly and/or in the direction of the second end of the membrane part and/or in the direction of the stent part. Alternatively or in addition, the guide can extend between the first end and the second end of the membrane part. Preferably, the thread's point of exit from the device is located closer to the second end of the membrane part than to the first end of the membrane part and/or it is located adjacent to the stent part.

The thread is arranged within the guide. It forms a loop around the opening at the first end of the membrane part and/or the thread completely or partially encircles the opening. The thread also extends between the first end and the second end. The thread exits the device at one place. Part of the thread is thus exposed and accessible. It is preferred that the thread exits the guide at the second end of the membrane part and/or near the base of the stent.

According to an embodiment, the membrane part comprises a lumen that extends between two opposing ends of the membrane part (first and second end), wherein each of said ends has an opening that connects with and allows for fluid flow through said lumen. In particular, the membrane part provides a passage (for the fluid transported by the vessel, preferably blood) that extends between said first and second end. The opening at the first end (the "flow opening") preferably provides the smallest cross-section along the passage and/or determines the flow rate through the passage.

According to one aspect, the flow opening constitutes the only opening or the largest opening of the device allowing the fluid to flow past the device.

The rigid member extends between the first end and the second end of the membrane part and/or between the flow opening and the stent part.

The member is "rigid" in the sense that it is more rigid than the membrane part. There can be one, two, three or more such rigid members.

The rigid member preferably extends along the guide and/or between the first end and the second end of the membrane part.

In particular, the rigid member is a tube, preferably made of metal or plastic, wherein the tube forms the part of the guide extending between the first end and the second end of the membrane part. In this case the thread can be movably arranged within the tube. If there is more than one rigid member, of course only one of them could be in the form of a tube to act as guide for the thread.

The rigid member can help centering the flow opening at the first end of the membrane part within the vessel and/or within the stent part. In particular, the rigid member can help prevent the first end of the membrane part from being drawn in the direction of the second end and/or in the direction of the stent when the thread is pulled on.

According to an aspect the thread is movable relative to the guide into a first direction to reduce the size of the flow opening (and/or to tighten the loop of the thread) and into a second direction to increase the size of the flow opening (and/or to enlarge the loop of the thread).

For example, the force required to move the thread into the second direction can be equal or higher than the force required to move the thread into the first direction.

It is preferred that the minimum force required to move the thread into the second direction is higher than 0.3, 0.4 or 0.5 and/or lower than 5, 3 or 1 Newton.

Alternatively or in addition the minimum force required to move the thread into the second direction can be higher than the maximum force exerted by the fluid on the device.

The adjustment of the required force can be achieved in different ways. For example, a narrowing of the guide can create increased friction between the guide and the thread (compared to the average friction between the guide and the thread).

According to one aspect the rigid member in the form of a tube has a coarctation that holds the thread. The degree of coarctation is determined in such a way that the force required for the movement of the thread past the coarctation is preferably more than 0.3 or 0.5 and/or less than 1.5 or 1 Newton. This allows for the thread to move in the tube during banding and reliable fixation of the thread in the tube after the banding.

At one point the thread can be fixed and/or attached to the rigid member and/or to the guide, for example by a narrowing of the guide or the tube (if the rigid member is a tube). Consequently, it is not only possible that both ends of the thread exit the device and/or the guide but also that only one end of the thread exits the device and/or the guide. According to one embodiment the loop which the thread forms can be essentially P-shaped with only one end of the thread exiting.

The function of the stent part is to hold the device in a fixed position relative to the vessel and/or to secure the device in the vessel and/or to secure the device to the wall of the vessel.

The stent part is adapted to contact an inner surface of the vessel for securing the device inside the vessel and/or to the inner surface of the vessel. Preferably, the stent part is held in place by exerting a radially outward pressure on the vessel.

For example, the stent part can be expandable and/or can have an adjustable diameter wherein the maximum diameter of the stent part is preferably larger than the diameter of the vessel into which the device is to be implanted.

Alternatively or in addition the diameter of the stent part can be more than 0.5, 1, or 2 and/or less than 4 or 3 Millimeters larger than the diameter of the vessel into which the stent is implanted.

According to one aspect, the stent part is adapted to sealably connect along its outer surface and/or along its outer circumference to the inner surface of the vessel to avoid fluid to pass between the stent part and the vessel surface.

The length of the stent part can preferably be more than 30, 40 or 50 and/or less than 150, 120 or 100 percent of the diameter of the stent part.

The stent part is connected to the membrane part, preferably at one end of the stent part and/or membrane part. The membrane part and the stent part can (from where they are connected) extend into opposite directions or into the same direction (preferably the direction of fluid flow), wherein the latter is preferred.

According to an aspect of the invention the membrane part is arranged within the stent part and/or an outer surface of the tube-shaped membrane part faces an inner surface of the stent part.

Preferably, the membrane part is made of a tube-shaped membrane, wherein the membrane is preferably made of polytetrafluoroethylene.

It is useful if the membrane is folded at the first end of the membrane part to form two layers, an outer layer of the membrane part and an inner layer of the membrane part. In this case the guide and/or the thread can be arranged between the two layers. For example, the surfaces of the two layers facing one another can form the guide for the thread. In particular, the part of the guide extending along the first end (preferably along and/or adjacent to the opening at the first end) of the membrane part and/or the part of the guide that extends between the first end and the second end of the membrane part can be formed in this manner. According to an aspect of the invention the rigid member is also arranged between the two layers of the membrane.

The membrane advantageously forms the connection between the membrane part and the stent part. For example, the membrane can continue from the membrane part to the stent part, wherein the membrane preferably forms a layer on the outside of the stent part facing away from the membrane part. Alternatively or additionally the membrane can be wrapped around the thread along the first end of the membrane part and/or wrapped around the stent.

A method for controlling the flow of a fluid (preferably blood) through a vessel and/or for adjustably reducing a vessel lumen can comprise pulling on a thread forming a loop around a flow opening of a device positioned in the vessel, thereby tightening the loop and reducing the size of the flow opening. Preferably, such a method makes use of the device described in this document.

If necessary, the diameter of the flow opening can be increased, for example by introducing an object (which is preferably not part of the device) into the flow opening and/or by applying pressure in a radially outward direction on the edge of the flow opening. The object can for example be a balloon and by inflating the balloon the flow opening can be enlarged. Other methods are however possible. For example, if the thread is sufficiently rigid it may be pushed into the device (and/or it may be pushed in the direction opposite to the direction it was pulled to reduce the diameter of the opening) thereby increasing the diameter of the flow opening. Such a step is also helped by the presence of a rigid member as described in this document.

The device can be introduced into a vessel and/or guided to (for example along the vessel) and positioned at a predetermined position within a vessel. Preferably this is done using a catheter. In this case the thread can be pulled out through the catheter.

Preferably, before pulling on the thread a catheter is placed adjacent to the point of exit of the thread from the device to stabilize the device.

The point of exit of the thread from the device is preferably located at the second end of the membrane part. In particular, the point of exit can be formed by the guide and/or the tube (if the rigid member is a tube).

After pulling on the thread and/or after the loop has been tightened and the diameter of the opening at the first end of the membrane part has been reduced the part of the thread exiting the device can be cut away, for example using a cutting catheter, wherein the cutting catheter preferably comprises a spiral tube and the thread is positioned between the turns of the spiral of the catheter for fixation of the thread while the part of the thread is cut off.

The device is intended for intravascular reduction of the vessel lumen, and can be used in medical conditions that require reduction of a blood flow and/and or reduction of the pressure distal from the site of device implantation.

The device is intended for adjustable reduction of the lumen of a vessel by endovascular access. It can be used for congenital and acquired heart defects when pulmonary artery banding is indicated. The device should be used in specialized hospitals that provide cardiovascular care for children and adults.

According to one embodiment the device comprises a stent, a membrane, a tube and a thread. Reduction of the vessel lumen is achieved with the help of the membrane. The stent is preferably not involved in reduction of the vessel lumen, it provides a fixation function for the whole device. The device enables to significantly reduce the diameter and the length of the device, as compared to devices that use metal parts of a stent to reduce the lumen.

According to one aspect the device comprises an outer part of the device (preferably the described stent part) and an inner part of the device (preferably the described membrane part). The outer part is adapted to contact the vessel and to anchor the device and/or secure it in place within the vessel, whereas the inner part has an opening (preferably the described flow opening) the size of which can be adjusted. The inner part is ring- or tube-shaped, wherein one end (preferably the described second end) and/or a base portion of the ring or tube is attached to the outer part of the device and an opposing end(preferably the described first end) and/or a front portion of the ring or tube comprises the opening. Preferably, the opening is the only and/or largest opening through which the fluid (preferably blood) which is transported by the vessel can pass to get from one (upstream) side of the device to the other (downstream) side. The size of the opening can be reduced by pulling at a thread that partially or completely encircles the opening, preferably arranged in the form of a loop. The thread is arranged within the inner part of the device and exits the device preferably at the base portion close to where it is connected to the outer part so that the exit point is close to the wall of the vessel. The inner part is flexible and/or made of a membrane.

The device described in this document is preferably a medical device for application in a vessel (in particular a blood vessel), for example a vessel of the body of a living animal or a living human being, in particular a human child and/or an adolescent human. The device can be used to control the flow of a fluid (in particular blood) through a vessel (in particular a blood vessel) and/or to adjustably reduce a vessel lumen (in particular by endovascular access) and/or for the treatment of a medical condition that requires the reduction of fluid flow and/or fluid pressure (in particular distal to and/or downstream of the site of device application). Disclosed is also a method for the aforementioned purpose(s) in which a device of the kind described in this document is used.

Preferably, in the context of this document the word "distal" means downstream of the device in respect of the flow of fluid (in particular blood).

According to a preferred definition, the direction perpendicular to a radial direction of the stent part and/or of the membrane part is defined as the "axial direction" of the device.

If the device or its parts are mentioned and if it is not clear from the context which state is meant preferably the device in its implanted state (inside a vessel) and alternatively in its non-implanted state (outside the vessel) shall be disclosed.

According to a preferred embodiment the device comprises a stent part providing a flow channel for the fluid and a flexible membrane part within the stent part that closes the flow channel off except for a flow opening. A thread is arranged within the membrane part with one end exiting the device and thus being accessible. The thread forms a loop around the flow opening and by pulling on the thread the loop can be constricted which leads to a reduction of the size of the flow opening.

### BRIEF DESCRIPTION OF THE DRAWINGS

FIG. **1A** is a lateral view of the device; the part of the membrane creating banding is closed. FIG. **1B** is a front cross section view of FIG. **1A****.**
FIG. **2A** is a lateral view of the device; the part of the membrane creating banding is opened. FIG. **2B** is a front cross section view of FIG. **2A****.**
FIG. **3** is a cross section of the proximal part of the device; the part of the membrane creating banding is closed.
FIG. **4** is an enlarged partial view of the cross section of the proximal part of the device; the part of the membrane creating banding is opened.
FIG. **5** shows the device installed as a flow restrictor in the main pulmonary artery.
FIG. **6** is a cross section view of a cutting catheter.

### DETAILED DESCRIPTION OF THE DRAWINGS

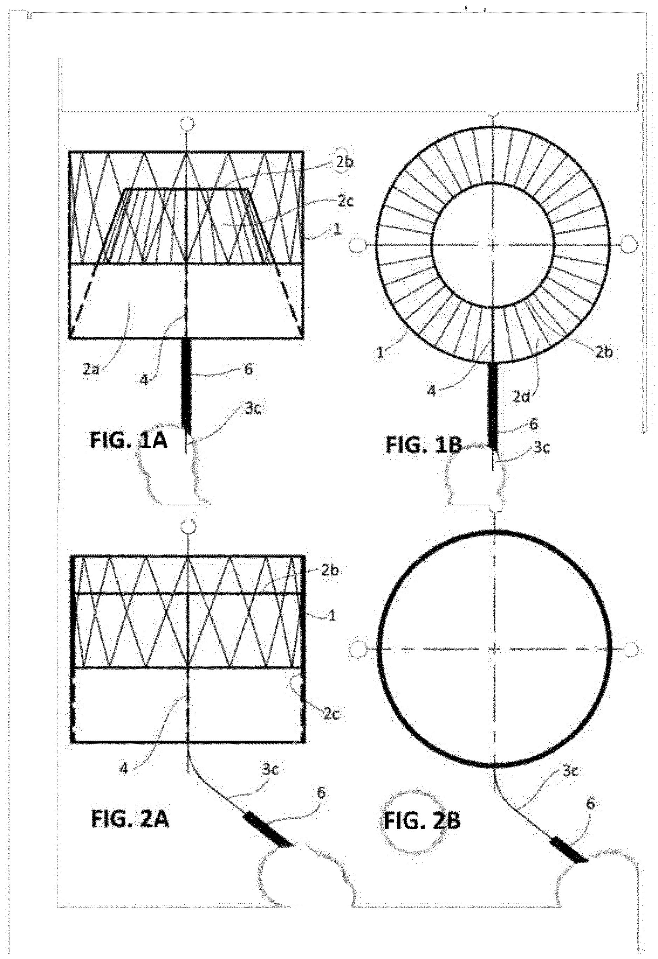

In the following a preferred embodiment of the invention is described. All figures 1A through 6 show the same embodiment.

The depicted device comprises a stent **1** (forming a stent part of the device), a membrane **2** (forming a membrane part of the device) and a thread **3.**

In all figures the stent **1** is opened and the proximal part of the stent **1** is partially covered by a layer **2a** of the membrane. The part of the membrane creating the banding (i.e. a restriction of the blood flow) is the free edge **2b** of the membrane which can be closed (as shown in FIG. **1A** - FIG. **1B**) or opened, as shown in FIG. **2A** - FIG. **2B.** Correspondingly, the opening provided by the device for the blood to pass which is defined by the free edge **2b** of the membrane is larger in the described opened state and smaller in the closed state. In the opened position the device does not hinder the blood flow, as the free edge **2b** of the membrane is placed along the stent.

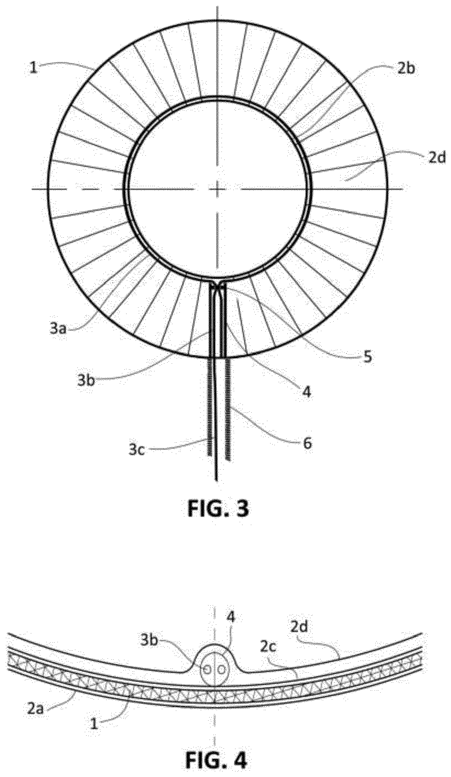

The membrane **2** is a one-piece tube that is rolled twice: once inside the stent **1** and once around the loop-forming part **3a** of the thread (see FIG. **3** - FIG. **4**). Thus, there is one membrane layer **2a** outside the stent and two membrane layers **2c, 2d** inside of it: an external layer **2c** and an internal layer **2d.** The loop forming part **3a** of the thread and a metal tube **4** are arranged between the internal **2d** and the external **2c** layer.

The thread **3** has three parts: a part that forms a loop **3a;** a fixation part **3b** inside the tube **4;** an adjusting part **3c** that is exposed and thus accessible.

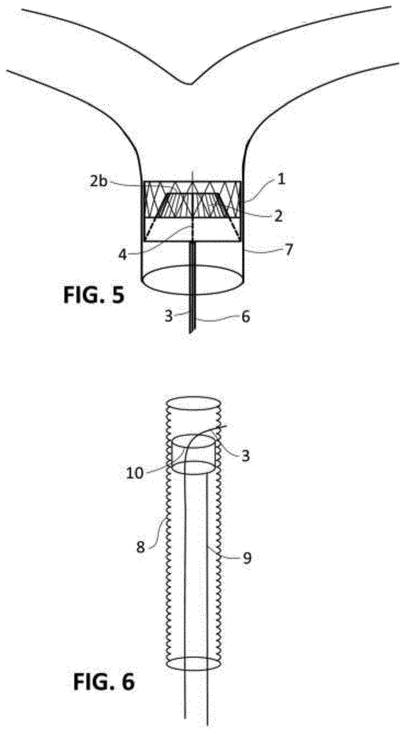

The device is placed in the trunk of the pulmonary artery **7** above the sinotubular junction (FIG. **5**). The banding is achieved by tightening the loop of the thread **3,** which leads to a reduction of the diameter of the free membrane edge **2b.** In order to prevent stent dislocation during traction, the stabilizing catheter **6** is placed right up to the tube **4.** The thread **3** can be pulled out through the stabilizing catheter **6** (FIG. **3**).

The necessary degree of banding is achieved under pressure control in the right ventricle and the pulmonary artery. If necessary the lumen can be increased by balloon dilatation. Increase and decrease of the vessel lumen can be performed repeatedly. Once the best diameter of banding is achieved, the thread **3** is cut off at the base of the stent **1** with a special cutting catheter (FIG. **6**). The cutting catheter is a spiral tube **8,** comprised of an elastic wire and a wire **9** with a sharp tube **10** at the end. The thread **3** is fixed among spiral turns and cut off with the help of the sharp tube **10.**

The inner diameter of the membrane for banding remains stable due to fixation of the thread **3b** in the tube **4** with coarctation **5** (FIG. **3**). After the adjusting part of the thread **3c** has been removed, it is impossible to further reduce the lumen. The increase of the lumen is possible at any time by balloon dilatation.

### NOMENCLATURE

- 1: stent
- 2: membrane
- 2a: layer of membrane outside the stent
- 2b: free edge of membrane
- 2c: external layer of membrane inside the stent
- 2d: internal layer of membrane inside the stent
- 3: thread
- 3a: loop forming part of the thread
- 3b: fixation part of the thread
- 3c: adjusting part of the thread
- 4: metal tube
- 5: coarctation
- 6: stabilizing catheter
- 7: pulmonary artery
- 8: spiral tube of cutting catheter
- 9: wire of cutting catheter
- 10: sharp tube of cutting catheter

## Claims

1. A medical device for controlling the flow of a fluid through a vessel comprising a stent part (1) adapted to contact an inner surface of the vessel and a membrane part (2) attached to the stent part (1), wherein the membrane part (2) forms a flow opening the size of which is adjustable by means of a thread (3) which extends along the flow opening, wherein
- the membrane part (2) is in the form of a tube with two openings at two opposing ends, wherein one of the two openings is the flow opening, wherein the two opposing ends comprise a first free end (2b) and a second end, wherein the flow opening is located at the first end (2b) and the second end is attached to the stent part (1), wherein the stent part (1) is preferably tube-shaped and the membrane part (2) is arranged within the stent part (1), **characterized in that**
- the membrane part (2) comprises a guide which extends along the flow opening and between the first end (2a) and the second end,
- a member extends between the first end (2a) and the second end of the membrane part (2), wherein the member is more rigid than the membrane part (2), and
- the thread (3) is arranged within the guide, wherein at the first end (2a) of the membrane part (2) the thread (3) forms a loop (3a) around the flow opening, extends between the first end (2a) and the second end of the membrane part (2), and exits the guide at the second end of the membrane part (2), and
- the size of the flow opening is adjustable by adjusting the size of the loop (3a) and wherein the size of the loop (3a) is adjustable by pulling on the thread (3) exiting the guide at the second end of the membrane part (2).

2. The medical device according to claim 1, wherein
- the member extends along the guide between the first end (2a) and the second end of the membrane part (2), or
- the member is a tube (4), preferably made of metal, wherein the tube (4) forms the part of the guide extending between the first end (2a) and the second end of the membrane part (2) and the thread (3) is arranged within the tube (4).

3. The medical device according to one of claims 1 to 2, wherein the thread (3) is movable relative to the guide into a first direction to tighten the loop (3a) and into a second direction to enlarge the loop (3a).

4. The medical device according to claim 3, wherein the minimum force required to move the thread (3) into the second direction is higher than the maximum force exerted by the fluid on the device and/or higher than 0.4 Newtons, wherein for this purpose the device preferably comprises means for increasing the friction between the guide and the thread (3), for example a narrowing of the guide exerting pressure on the thread (3).

5. The medical device according to one of claims 1 to 4, wherein
- the membrane part (2) is made of a tube-shaped membrane, wherein the membrane is preferably made of polytetrafluoroethylene,
- the membrane is folded along the first end of the membrane part (2) to form two layers, an outer layer (2c) of the membrane part (2) facing the stent part (1) and an inner layer (2d) of the membrane part (2), and
- the part of the guide extending along the flow opening at the first end (2a) of the membrane part (2) is formed by a space between the outer and the inner layer (2c, 2d) of the membrane part (2).

6. The medical device according to claim 5, wherein the membrane continues from the membrane part (2) to the stent part (1) forming the connection between the stent part (1) and the membrane part (2), wherein the membrane preferably forms a layer on the outside of the stent part (1) facing away from the membrane part (2).

7. The medical device according to one of claims 5 to 6, wherein the membrane is wrapped around the thread (3) along the first end (2a) of the membrane part (2) and also wrapped around the stent part (1).

## Patentansprüche

1. Medizinische Vorrichtung zum Regeln des Stroms eines Fluids durch ein Gefäß, die einen Stentteil (1), der angepasst ist, um eine innere Fläche des Gefäßes zu berühren, und einen Membranteil (2), der an dem Stentteil (1) befestigt ist, umfasst, wobei der Membranteil (2) eine Durchflussöffnung bildet, deren Größe mit Hilfe eines Fadens (3) einstellbar ist, der sich entlang der Durchflussöffnung erstreckt, wobei
- der Membranteil (2) in der Form einer Röhre mit zwei Öffnungen an zwei entgegengesetzten Enden vorliegt, wobei eine der zwei Öffnungen die Durchflussöffnung ist, wobei die zwei entgegengesetzten Enden ein erstes freies Ende (2b) und ein zweites Ende umfassen, wobei die Durchflussöffnung an dem ersten Ende (2b) angeordnet ist und das zweite Ende an dem Stentteil (1) befestigt ist, wobei der Stentteil (1) vorzugsweise röhrenförmig ist und der Membranteil (2) innerhalb des Stentteils (1) angeordnet ist, **dadurch gekennzeichnet, dass**
- der Membranteil (2) eine Führung umfasst, die sich entlang der Durchflussöffnung und zwischen dem ersten Ende (2a) und dem zweiten Ende erstreckt,
- sich ein Element zwischen dem ersten Ende (2a) und dem zweiten Ende erstreckt, wobei das Element steifer ist als der Membranteil (2), und
- der Faden (3) innerhalb der Führung angeordnet ist, wobei der Faden (3) an dem ersten Ende (2a) des Membranteils (2) eine Schlinge (3a) um die Durchflussöffnung bildet, sich zwischen dem ersten Ende (2a) und dem zweiten Ende des Membranteils (2) erstreckt und an dem zweiten Ende des Membranteils (2) aus der Führung austritt, und
- die Größe der Durchflussöffnung durch Einstellen der Größe der Schlinge (3a) einstellbar ist und wobei die Größe der Schlinge (3a) durch Ziehen an dem Faden (3), der an dem zweiten Ende des Membranteils (2) aus der Führung austritt, einstellbar ist.

2. Medizinische Vorrichtung nach Anspruch 1, wobei
- sich das Element entlang der Führung zwischen dem ersten Ende (2a) und dem zweiten Ende des Membranteils (2) erstreckt oder
- das Element eine Röhre (4) ist, die vorzugsweise aus Metall hergestellt ist, wobei die Röhre (4) den Teil der Führung bildet, der sich zwischen dem ersten Ende (2a) und dem zweiten Ende des Membranteils (2) erstreckt, und der Faden (3) innerhalb der Röhre (4) angeordnet ist.

3. Medizinische Vorrichtung nach einem der Ansprüche 1 bis 2, wobei der Faden (3) im Verhältnis zu der Führung in eine erste Richtung, um die Schlinge (3a) zusammenzuziehen, und in eine zweite Richtung, um die Schlinge (3a) zu vergrößern, beweglich ist.

4. Medizinische Vorrichtung nach Anspruch 3, wobei die minimale Kraft, die erforderlich ist, um den Faden (3) in die zweite Richtung zu bewegen, größer ist als die maximale Kraft, die durch das Fluid auf die Vorrichtung ausgeübt wird, und/oder größer als 0,4 Newton, wobei die Vorrichtung zu diesem Zweck vorzugsweise Mittel zum Steigern der Reibung zwischen der Führung und dem Faden (3), zum Beispiel eine Verengung der Führung, die Druck auf den Faden (3) ausübt, umfasst.

5. Medizinische Vorrichtung nach einem der Ansprüche 1 bis 4, wobei
- der Membranteil (2) aus einer röhrenförmigen Membran hergestellt ist, wobei die Membran vorzugsweise aus Polytetrafluorethylen hergestellt ist,
- die Membran entlang des ersten Endes des Membranteils (2) gefaltet ist, um zwei Lagen, eine äußere Lage (2c) des Membranteils (2), die dem Stentteil (1) gegenüberliegt, und eine innere Lage (2d) des Membranteils (2), zu bilden, und
- der Teil der Führung, der sich entlang der Durchflussöffnung an dem ersten Ende (2a) des Membranteils (2) erstreckt, durch einen Raum zwischen der äußeren und der inneren Lage (2c, 2d) des Membranteils (2) gebildet wird.

6. Medizinische Vorrichtung nach Anspruch 5, wobei sich die Membran von dem Membranteil (2) bis zu dem Stentteil (1) fortsetzt, wobei sie die Verbindung zwischen dem Stentteil (1) und dem Membranteil (2) bildet, wobei die Membran vorzugsweise eine Lage auf der Außenseite des Stentteils (1), die von dem Membranteil (2) weg zeigt, bildet.

7. Medizinische Vorrichtung nach einem der Ansprüche 5 bis 6, wobei die Membran entlang des ersten Endes (2a) des Membranteils (2) um den Faden (3) gewickelt ist und ebenfalls um den Stentteil (1) gewickelt ist.

## Revendications

1. Dispositif médical de commande d'écoulement d'un fluide à travers un vaisseau comprenant une partie de stent (1) adaptée pour contacter une surface intérieure du vaisseau et une partie de membrane (2) fixée à la partie de stent (1), dans lequel la partie de membrane (2) forme une ouverture d'écoulement dont la taille est réglable au moyen d'un filetage (3) qui s'étend le long de l'ouverture d'écoulement, dans lequel
- la partie de membrane (2) a la forme d'un tube avec deux ouvertures à deux extrémités opposées, dans lequel une des deux ouvertures est l'ouverture d'écoulement, dans lequel les deux extrémités opposées comprennent une première extrémité libre (2b) et une seconde extrémité, dans lequel l'ouverture d'écoulement est située sur la première extrémité (2b) et la seconde extrémité est fixée sur la partie de stent (1), dans lequel la partie de stent (1) est de préférence tubulaire et la partie de membrane (2) est agencée à l'intérieur de la partie de stent (1), **caractérisé en ce que**
- la partie de membrane (2) comprend un guide qui s'étend le long de l'ouverture d'écoulement et entre la première extrémité (2a) et la seconde extrémité,
- un membre s'étend entre la première extrémité (2a) et la seconde extrémité de la partie de membrane (2), dans lequel le membre est plus rigide que la partie de membrane (2), et
- le filetage (3) est agencé à l'intérieur du guide, dans lequel à la première extrémité (2a) de la partie de membrane (2), le filetage (3) forme une boucle (3a) autour de l'ouverture d'écoulement, s'étend entre la première extrémité (2a) et la seconde extrémité de la partie de membrane (2) et sort du guide à la seconde extrémité de la partie de membrane (2), et
- la taille de l'ouverture d'écoulement est réglable en ajustant la taille de la boucle (3a) et dans lequel la taille de la boucle (3a) est ajustable en tirant sur le filetage (3) sortant du guide à la seconde extrémité de la partie de membrane (2).

2. Dispositif médical selon la revendication 1, dans lequel
- le membre s'étend le long du guide entre la première extrémité (2a) et la seconde extrémité de la partie de membrane (2), ou
- le membre est un tube (4), de préférence en métal, dans lequel le tube (4) forme la partie du guide s'étendant entre la première extrémité (2a) et la seconde extrémité de la partie de membrane (2) et le filetage (3) est agencé à l'intérieur du tube (4).

3. Dispositif médical selon l'une des revendications 1 à 2, dans lequel le filetage (3) est mobile par rapport au guide dans une première direction pour resserrer la boucle (3a) et dans une seconde direction pour élargir la boucle (3a).

4. Dispositif médical selon la revendication 3, dans lequel la force minimale requise pour déplacer le filetage (3) dans la seconde direction est supérieure à la force maximale exercée par le fluide sur le dispositif et/ou supérieure à 0,4 Newtons, dans lequel à cet effet, le dispositif comprend de préférence un moyen pour augmenter le frottement entre le guide et le filetage (3), par exemple un rétrécissement du guide exerçant de la pression sur le filetage (3).

5. Dispositif médical selon l'une des revendications 1 à 4, dans lequel
- la partie de membrane (2) est faite d'une membrane tubulaire, dans lequel la membrane est de préférence en polytétrafluoroéthylène,
- la membrane est pliée le long de la première extrémité de la partie de membrane (2) pour former deux couches, une couche extérieure (2c) de la partie de membrane (2) faisant face à la partie de stent (1) et une couche intérieure (2d) de la partie de membrane (2), et
- la partie du guide s'étendant le long de l'ouverture d'écoulement à la première extrémité (2a) de la partie de membrane (2) est formée par un espace entre la couche extérieure et intérieure (2c, 2d) de la partie de membrane (2).

6. Dispositif médical selon la revendication 5, dans lequel la membrane continue de la partie de membrane (2) vers la partie de stent (1) formant la liaison entre la partie de stent (1) et la partie de membrane (2), dans lequel la membrane forme de préférence une couche sur l'extérieur de la partie de stent (1) s'éloignant de la partie de membrane (2) .

7. Dispositif médical selon l'une des revendications 5 à 6, dans lequel la membrane est enveloppée autour du filetage (3) le long de la première extrémité (2a) de la partie de membrane (2) et également enveloppée autour de la partie de stent (1).
